Europäisches Patentamt

European Patent Office

Office européen des brevets

(19)

(11) Publication number: **0 487 729 A1**

## EUROPEAN PATENT APPLICATION
### published in accordance with Art. 158(3) EPC

(12)

(21) Application number: **90912030.5**

(22) Date of filing: **10.08.90**

(86) International application number:
**PCT/JP90/01023**

(87) International publication number:
**WO 91/02528 (07.03.91 91/06)**

(51) Int. Cl.⁵: **A61K 31/445**

(30) Priority: **18.08.89 JP 213876/89**

(43) Date of publication of application:
**03.06.92 Bulletin 92/23**

(84) Designated Contracting States:
**AT BE CH DE DK ES FR GB IT LI NL SE**

(71) Applicant: **HISAMITSU PHARMACEUTICAL CO., INC.**
**408, Tashirodaikan-machi**
**Tosu-shi Saga-ken(JP)**

(72) Inventor: **NAKAGAWA, Akira**
**970-11, Fujinokimachi**
**Tosu-shi Saga 841(JP)**
Inventor: **MIYATA, Satoru 103, Nishidamachi**
**Tosu-shi**
**Saga 841(JP)**

(74) Representative: **Silverman, Warren et al**
**HASELTINE LAKE & CO. Hazlitt House 28**
**Southampton Buildings Chancery Lane**
**London WC2A 1AT(GB)**

(54) **KETOTIFEN PREPARATION FOR EXTERNAL USE.**

(57) A ketotifen preparation for applying to the skin comprising as the active ingredient ketotifen or its fumarate and further containing as a stabilizer at least one compound selected from the group consisting of hydrogensulfites, sulfites, pyrosulfites, and dibutylhydroxytoluene.

EP 0 487 729 A1

Technical Field:

This invention relates to a preparation for external use which contains ketotifen or its fumarate in a stable state as the active ingredient. More particularly, it relates to a ketotifen preparation for external use containing ketotifen, which has been mainly used in oral preparations as an antiallergic agent, in a stable state and being useful as a skin preparation for external use for treating dermatological diseases such as dermatitis, eczema, atopic eczema and rash.

Background Art:

Ketotifen {4-(1-methyl-4-piperidylidene)-4H-benzo[4,5]cyclohepta[1,2-b]thiophen-10[9H]-one} is a drug having antiallergic effects over a wide range, for example, antihistamic and anti-SRS-A effects. Ketotifen fumarate has been commonly used as oral preparations. Examples of the prior art disclosing a preparation for external use containing ketotifen or its fumarate include J.P.A. Laid-Open Gazettes Nos. (Sho.)51-32724, (Sho.)51-142543, (Sho.)57-181007, (Sho.)60-190710, (Sho.)62-164624, (Sho.)62-223119, (Hei.)1-102024 and (Hei.)1-121218. However,the stability of such a preparation is fully taken into consideration in none of these references. That is to say, the conventional preparations for external use containing ketotifen or its fumarate decompose and discolor with the lapse of time. Thus no satisfactory preparation can be obtained thereby.

Although ketotifen or its fumarate is relatively stable in powdery form, the solution thereof is unstable and discolors when incorporated into a preparation. The discolored preparation is undesirable not only because of the poor appearance but also from the viewpoint of safety.

Accordingly, it is an object of the present invention to maintain the stability of ketotifen or its fumarate and to prevent any discoloration of the preparation containing the same.

Disclosure of Invention:

In order to maintain the stability of ketotifen or its fumarate in the preparation with the lapse of time, the present inventors have conducted extensive studies. As a result, they have successfully found out that the stability of ketotifen or its fumarate can be substantially improved by adding one or more compounds selected from the group consisting of hydrogensulfites, sulfites, pyrosulfites and dibutylhydroxytoluene, as a stabilizer, to the preparation for external use containing ketotifen or its fumarate. The present invention is based on this finding.

Examples of the dosage form of the preparation for external use of the present invention include cream, solution, lotion, gel, eye drops, aerosol, plaster, cataplasm, stomatic preparation and suppository.

Examples of the hydrogensulfite to be used as the stabilizer in the present invention include sodium hydrogensulfite, potassium hydrogensulfite and ammonium hydrogensulfite. Examples of the sulfite include sodium sulfite, potassium sulfite and barium sulfite. Examples of the pyrosulfite include sodium pyrosulfite and potassium pyrosulfite. Either one of these stabilizers or a combination thereof may be used. It is most desirable to use sodium hydrogensulfite, sodium sulfite, sodium pyrosulfite or a combination of at least one of these stabilizers and dibutylhydroxytoluene (BHT).

These stabilizers may be used in an amount of from 0.3 to 50 parts by weight, preferably from 2 to 20 parts by weight, per 100 parts by weight of ketotifen or its fumarate. When the content of the stabilizer is outside the range as specified above, i.e., excessively smaller or larger, no expected stabilizing effect tends to be achieved. When the content of the stabilizer is above the upper limit as specified above, in particular, there is a risk that the stabilizer would react with ketotifen and the preparation would turn pale brown in the production step.

The addition of BHT alone can exert only a limited stabilizing effect. Therefore, it is recommended to combine BHT with at least one stabilizer selected from the group consisting of the hydrogensulfites, sulfites and pyrosulfites so as to achieve an improved effect. In this case, it is preferable to use from 10 to 500 parts by weight of the sulfite compound(s) per 100 parts by weight of BHT.

When the present invention is to be embodied with the use of the hydrogensulfite, sulfite or pyrosulfite as the stabilizer, the stabilizer may be added in the form of a solution in purified water or as powder.

Best Mode for Carrying Out the Invention:

To further illustrate the present invention in greater detail, the following Examples will be given.

Example 1

0.5 g of ketotifen was mixed with 2 g of benzyl alcohol, 5 g of diisopropyl sebacate, 10 g of isopropyl myristate and 5 g of polyoxyethylene(20) sorbitan monostearate (TS-10; a product of Nikko Chemicals Co., Ltd.) and heated to 50°C to give a solution. Next, an aqueous phase, prepared by dissolving 5 g of 1,3-butylene glycol and 0.2 g of methylparaben in 65.88 g of purified water at 50°C, was added to the solution and the obtained mixture was emulsified at 50°C. The obtained solution was mixed with 0.8 g of carboxyvinyl polymer and cooled to room temperature under stirring. To the obtained solution was added another solution, prepared by dissolving 0.8 g of diisopropanolamine and 0.02 g of sodium hydrogensulfite in 10 g of purified water, and the mixture was stirred until a homogeneous state was achieved. Thus a cream containing ketotifen was obtained.

Example 2

0.3 g of ketotifen was mixed with 2 g of crotamiton, 5 g of 2-octyldodecanol, 10 g of isopropyl myristate, 5 g of polyoxyethylene(20) sorbitan monostearate (TS-10; a product of Nikko Chemicals Co., Ltd.) and 0.03 g of BHT and heated to 70°C to give a solution. Next, an aqueous phase, prepared by dissolving 5 g of 1,3-butylene glycol and 0.2 g of methylparaben in 60.84 g of purified water at 50°C, was added to the solution and the obtained mixture was emulsified at 50°C. The obtained solution was mixed with 0.8 g of carboxyvinyl polymer and cooled to room temperature under stirring. To the obtained solution was added another solution, prepared by dissolving 0.8 g of diisopropanolamine and 0.03 g of sodium hydrogensulfite in 10 g of purified water, and the mixture was stirred until a homogeneous state was achieved. Thus a cream containing ketotifen was obtained.

Example 3

0.3 g of ketotifen was dissolved in 1.5 g of benzyl alcohol by heating to 70°C. Next, 15 g of white petrolatum, 10 g of cetostearyl alcohol, polyoxyethylene(23) cetyl ether (BC-23, a product of Nikko Chemicals Co., Ltd.), 0.02 g of BHT and 3 g of liquid paraffin were dissolved in the obtained solution by heating to 75°C to thereby give an oily phase. Separately, 60.86 g of purified water was heated to 75°C and 0.3 g of methylparaben and 5 g of 1,3-butylene glycol were dissolved therein. The aqueous phase thus obtained was added to the oily phase and the resulting mixture was emulsified by stirring. After adding 0.02 g of sodium hydrogensulfite thereto, the mixture was further stirred and cooled to room temperature. Thus a cream containing ketotifen was obtained.

Example 4

0.2 g of ketotifen and 0.01 g of BHT were dissolved in 60 g of 1,3-butylene glycol by heating to 70°C. To the obtained solution were added 39.77 g of purified water and 0.02 g of sodium pyrosulfite, and then the obtained mixture was stirred. Thus a solution containing ketotifen was obtained.

Example 5

0.5 g of ketotifen was dissolved in 4 g of crotamiton by heating to 70°C. In the obtained solution were dissolved 85.45 g of white petrolatum, 5 g of glycerol monostearate and 5 g of beef tallow by heating to 70°C. Then 0.05 g of sodium hydrogensulfite was added to the resulting solution under stirring. The obtained mixture was further stirred and cooled to room temperature. Thus an ointment containing ketotifen was obtained.

Example 6

0.5 g of ketotifen was dissolved in 2 g of diisopropyl sebacate and 50 g of 1,3-butylene glycol by heating to 70°C. To the obtained solution was added another solution of 0.7 g of carboxyvinyl polymer swelling in 36.27 g of purified water, and then the obtained mixture was stirred. Next, to the mixed solution was added a solution, prepared by dissolving 0.03 g of sodium hydrogensulfite and 0.5 g of diisopropanolamine in 10 g of purified water, and then the obtained mixture was stirred. Thus a gel containing ketotifen was obtained.

Example 7

0.3 g of ketotifen was mixed with 2 g of crotamiton, 5 g of 2-octyldodecanol, 5 g of isopropyl myristate, 5 g of polyoxyethylene (20) sorbitan monostearate (TS-10; a product of Nikko Chemicals Co., Ltd.), 0.5 g of cholesterol and 0.03 g of BHT and heated to 70°C to give a solution. Next, an aqueous phase, prepared by dissolving 5 g of 1,3-butylene glycol and 0.2 g of methylparaben in 66.74 g of purified water at 50°C, was added to the solution and the obtained mixture was cooled to room temperature under stirring. To the obtained solution was added another solution, prepared by dissolving 0.2 g of diisopropanolamine and 0.03 g of sodium hydrogensulfite in 10 g of purified water, and the obtained mixture was stirred until a homogeneous state was achieved. Thus a lotion containing ketotifen was obtained.

Example 8

0.05 g of benzalkonium chloride and 2.5 g of chlorobutanol were dissolved in 800 ml of distilled water by heating. Next, 2.84 g of sodium dihydrogenphosphate, 5.6 g of disodium hydrogenphosphate and 0.2 g of sodium hydrogensulfite were added to the obtained solution and the total volume was adjusted to 1,000 ml with distilled water. After filtering through a filter paper, the obtained filtrate was steam-sterilized under elevated pressure at 120°C for 30 minutes and stored. In a portion of the aqueous solution thus obtained were dissolved 0.1 g of ketotifen fumarate and 0.418 g of sodium chloride. Then the total volume of the resulting mixture was adjusted to 100 ml with the aqueous solution. After filtering through a membrane filter (0.22 $\mu$m), the filtrate was pipetted into eye drop containers. Thus eye drops containing ketotifen were obtained.

Example 9

0.5 g of ketotifen was dissolved in 4 g of crotamiton by heating to 70°C. In the obtained solution were dissolved 85.3 g of white petrolatum, 5 g of glycerol monostearate and 5 g of beef tallow by heating to 70°C. Then 0.2 g of sodium hydrogensulfite was added to the resulting solution under stirring. The obtained mixture was further stirred and cooled to room temperature. Thus an ointment containing ketotifen was obtained.

Example 10

A mixture of 30.5 parts by weight of a styrene/isoprene/styrene block copolymer (Cariflex TR-1107; a product of Shell Chemicals Co.), 18.0 parts by weight of liquid paraffin and 46.0 parts by weight of Arkon P-85 (a product of Arakawa Kagaku Co., Ltd.), employed as an adhesion-increasing agent (adhesive), was heated to give a solution. Then a mixture consisting of 0.1 part by weight of ketotifen fumarate, 5.5 parts by weight of mentha oil, employed as an absorption promoter, and 0.03 parts by weight of sodium hydrogensulfite was mixed with the solution. The obtained mixture was spread over a silicone-treated polyester film in a thickness of 100 $\mu$m. After being covered with an aluminum-metallized nylon/polyester nonwoven fabric, the film was calendered, transferred and cut into pieces of a desired size. Thus a ketotifen-containing tape of the present invention was obtained. The obtained tape could liberate the active ingredient at a high efficiency, showed an extremely high extent of bioavailability and never irritated the skin.

Example 11

A mixture of 30.0 parts by weight of a styrene/isoprene/styrene block copolymer (Cariflex TR-1107; a product of Shell Chemicals Co.), 15.0 parts by weight of liquid paraffin and 46.0 parts by weight of Arkon (a product of Arakawa Kagaku Co., Ltd.), employed as an adhesion-increasing agent, was heated to give a solution. Then 0.2 parts by weight of ketotifen and 0.02 parts by weight of sodium hydrogensulfite dissolved in 5.0 parts by weight of mentha oil, employed as an absorption promoter, were mixed with the solution. The obtained mixture was spread over a polyester film-laminated vinyl choloride film in a thickness of 100 $\mu$m. After being covered with a silicone-treated polyester film, the film was cut into pieces of a desired size. Thus a ketotifen-containing tape of the present invention was obtained. The obtained tape showed excellent properties, similar to the one of the Example 10.

Example 12

A mixture of 22.5 parts by weight of a styrene/isoprene/styrene block copolymer (Cariflex TR-1111; a product of Shell Chemicals Co.), 35.5 parts by weight of liquid paraffin and 34.5 parts by weight of YS-Resin (a product of Yasuhara Yushi Co., Ltd.), employed as an adhesion-increasing agent, was heated to give a solution. Then 0.1 part by weight of ketotifen dissolved in 5.0 parts by weitht of mentha oil, employed as an absorption promoter, and 0.02 parts by weight of sodium hydrogensulfite were mixed with the solution. The obtained mixture was spread over a silicon-treated polyester film in a thickness of 120 μm. After being covered with a polyethylene film, the film was calendered, transferred and cut into pieces of a desired size. Thus a ketotifen-containing tape of the present invention was obtained. The obtained tape showed excellent properties, similar to the one of the Example 10.

Example 13

A mixture of 30.0 parts by weight of a styrene/isoprene/styrene block copolymer (Solprene 418; a product of Phillip Petroleum Co.), 20.0 parts by weight of liquid paraffin and 36.0 parts by weight of Quintone (a product of Nippon Zeon Co., Ltd.), employed as an adhesion-increasing agent, was heated to give a solution. Then 0.3 part by weight of ketotifen fumarate, 0.3 parts by weight of mentha oil, employed as an absorption promoter, and 0.02 part by weight of sodium pyrosulfite were mixed with the solution. The obtained mixture was spread over a polypropylene film in a thickness of 80 μm. After being covered with a silicone-treated polyester film, the film was cut into pieces of a desired size. Thus a ketotifen-containing tape of the present invention was obtained. The obtained tape showed excellent properties, similar to the one of the Example 10.

Comparative Example 1

0.5 g of ketotifen was mixed with 2 g of benzyl alcohol, 5 g of diisopropyl sebacate, 10 g of isopropyl myristate and 5 g of polyoxyethylene(20) sorbitan monostearate (TS-10; a product of Nikko Chemicals Co., Ltd.) and heated to 50°C to give a solution. Next, an aqueous phase, prepared by dissolving 5 g of 1,3-butylene glycol and 0.2 g of methylparaben in 61 g of purified water at 50°C, was added to the solution and the obtained mixture was emulsified at 50°C. To the obtained solution was added 0.8 g of carboxyvinyl polymer and cooled to room temperature under stirring. To the resulting solution was added another solution, prepared by dissolving 0.8 g of diisopropanolamine in 10 g of purified water, and stirred until a homogeneous state was achieved. Thus a cream containing ketotifen was obtained.

Comparative Example 2

0.5 g of ketotifen was mixed with 2 g of benzyl alcohol, 5 g of diisopropyl sebacate, 10 g of isopropyl myristate, 5 g of polyoxyethylene(20) sorbitan monostearate (TS-10; a product of Nikko Chemicals Co., Ltd.) and 0.1 g of BHT and heated to 50°C to give a solution. Next, an aqueous phase, prepared by dissolving 5 g of 1,3-butylene glycol and 0.2 g of methylparaben in 61 g of purified water at 50°C, was added to the solution and the obtained mixture was emulsified at 50°C. To the obtained solution was added 0.8 g of carboxyvinyl polymer and cooled to room temperature under stirring. To the resulting solution was added another solution, prepared by dissolving 0.8 g of diisopropanolamine in 10 g of purified water, and stirred until a homogeneous state was achieved. Thus a cream containing ketotifen was obtained.

Comparative Example 3

0.5 g of ketotifen was mixed with 2 g of benzyl alcohol, 5 g of diisopropyl sebacate, 10 g of isopropyl myristate and 5 g of polyoxyethylene(20) sorbitan monostearate (TS-10; a product of Nikko Chemicals Co., Ltd.) and heated to 50°C to give a solution. Next, an aqueous phase, prepared by dissolving 5 g of 1,3-butylene glycol and 0.2 g of methylparaben in 61 g of purified water at 50°C, was added to the solution and the obtained mixture was emulsified at 50°C. To the obtained solution was added 0.8 g of carboxyvinyl polymer and cooled to room temperature under stirring. To the resulting solution was added another solution, prepared by dissolving 0.8 g of diisopropanolamine and 0.2 g of sodium hydrogensulfite in 10 g of purified water, and stirred until a homogeneous state was achieved. Thus a cream containing ketotifen was obtained.

Experiment 1 (Stability test):

The creams prepared in the Examples 1 and 2 and the Comparative Examples 1, 2 and 3 were each packed in a tube and stored at 60°C to observe a change in the appearance of each cream. Table 1 summarizes the results.

Table 1

| Stability test of ketotifen creams (60°C) | | | |
|---|---|---|---|
| | Immediately after production | After 15 days | After 30 days |
| Ex. 1 | faintly yellow white | faintly yellow white | faintly yellow white |
| Ex. 2 | faintly yellow white | faintly yellow white | faintly yellow white |
| Comp. Ex. 1 | faintly yellow white | yellow | brown |
| Comp. Ex. 2 | white | yellow | yellow |
| Comp. Ex. 3 | faintly yellow white | faintly brown | brown |

As apparent from the results indicated in Table 1, the creams of the present invention each containing an appropriate amount of the stabilizer are superior in heat stability to the comparative ones.

Industrial Applicability:

The preparation of the present invention containing ketotifen or its fumarate in a stable state remains stable without causing any discoloration even after a prolonged storage. Therefore, the commercial value of the preparation can be maintained for a long time. Furthermore, the preparation of the present invention, in which no decomposition product is formed, is preferable from the viewpoint of safety.

These facts indicate that the preparation for external use of the present invention containing ketotifen or its fumarate in a stable state has a high industrial applicability as a remedy for skin diseases such as dermatitis, eczema, atopic dermatitis and rash.

**Claims**

1. A ketotifen preparation for external use comprising ketotifen or its fumarate as an active ingredient, which contains at least one member selected from the group consisting of hydrogensulfites, sulfites, pyrosulfites and dibutylhydroxytoluene as a stabilizer.

2. A ketotifen preparation for external use according to claim 1, wherein the content of said stabilizer ranges from 0.3 to 50 parts by weight per 100 parts by weight of ketotifen or its fumarate.

**Amended claims**

1. (amended) A ketotifen preparation for external use comprising ketotifen or its fumarate as as active ingredient, which contains at least one sulfite compound selected from the group consisting of sodium hydrogensulfite, potassium hydrogensulfite, sodium sulfite, potassium sulfite, sodium pyrosulfite and potassium pyrosulfite as a stabilizer.

2. A ketotifen preparation for external use according to claim 1, wherein the content of said stabilizer ranges from 0.3 to 50 parts by weight per 100 parts by weight of ketotifen or its fumarate.

3. (added) A ketotifen preparation for external use according to claim 1 or 2, which further contains dibutylhydroxytoluene as a stabilizer.

4. (added) A ketotifen preparation for external use according to claim 3, wherein the content of said sulfite compound ranges from 10 to 500 parts by weight per 100 parts by weight of dibutylhydroxytoluene.

# INTERNATIONAL SEARCH REPORT

International Application No PCT/JP90/01023

## I. CLASSIFICATION OF SUBJECT MATTER (if several classification symbols apply, indicate all) 6

According to International Patent Classification (IPC) or to both National Classification and IPC

Int. Cl$^5$     A61K31/445

## II. FIELDS SEARCHED

Minimum Documentation Searched 7

| Classification System | Classification Symbols |
|---|---|
| IPC | A61K31/445, A61K9/70, A61K9/06, A61K9/08, A61K9/107 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| Y | JP, A, 1-121218 (Ikeda Mohando K.K.), 12 May 1989 (12. 05. 89), Claim and upper right column page 4 (Family: none) | 1 - 2 |
| A | JP, A, 57-108011 (Toyo Ink Mfg. Co., Ltd.), 5 July 1982 (05. 07. 82), Lower column, page 3 (Family: none) | 1 - 2 |
| A | JP, A, 59-89621 (CIBA-Geigy AG), 23 May 1984 (23. 05. 84), Claim and right column to lower left column, page 9 & DE, A1, 3336047 & GB, A1, 2128087 | 1 - 2 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| October 19, 1990 (19. 10. 90) | November 5, 1990 (05. 11. 90) |
| International Searching Authority<br><br>Japanese Patent Office | Signature of Authorized Officer |

Form PCT/ISA/210 (second sheet) (January 1985)